# EUROPEAN PATENT APPLICATION

(11) **EP 3 231 776 A1**
(43) Date of publication of application: **18.10.2017**
(21) Application number: 15866495.3
(22) Date of filing: 08.12.2015
(51) Int. Cl.: C03C 13/00, A61F 13/00, A61L 15/16, C03B 37/06

(54) **GLASS COMPOSITION FOR WOUND HEALING, WOUND COVERING MATERIAL, AND METHOD FOR PRODUCING SAME**

(30) Priority: 11.12.2014 JP 2014250542
(71) Applicant: Nippon Electric Glass Co., Ltd., Otsu-shi Shiga 520-8639 (JP)
(72) Inventor: SAWASATO, Hiroshi, Otsu-shi Shiga 520-8639 (JP); CHOJU, Ken, Otsu-shi Shiga 520-8639 (JP)
(74) Representative: Henkel, Breuer & Partner
(86) International application number: PCT/JP2015/084358
(87) International publication number: WO 2016/093211

(57) **Abstract**

Provided are: a glass composition for wound healing, said composition promoting a wound healing process by providing the moist environment and nutrients required for growth of epidermal cells, and having bactericidal properties for preventing the critical fixing of bacteria to a wound surface and infection with said bacteria; and a wound covering material that uses said glass composition. The glass composition is characterized by containing, by mass% in terms of oxides, 5-70% of SiO₂, 0-10% of Al₂O₃, 5-40.0% of B₂O₃, and 1-50% of CaO.

## Description

### Technical Field

The present invention relates to a glass composition that exhibits an excellent healing effect for wound surfaces of cuts, lacerations, crush injuries, burns, bedsores and others, to a wound covering material produced using the composition and to its production method.

### Background Art

Heretofore, for wound care, the wound surface is first disinfected and then protected with gauze. However, in such a healing method, epidermal cells would die by disinfection. In addition, these days, it has become known that epidermal cells are difficult to grow on drying of wound surfaces.

Accordingly, Makoto Natsui, a plastic surgeon, et al. have propounded a moist wound healing method of promoting cell growth by keeping a wound surface in a moist environment, in place of treatment using disinfectant and gauze, and at present, this healing method has become widespread (Non Patent Literature 1).

Wound covering materials are also called wound care dressings, and are used for maintaining the moist environment of a wound surface in wound care. Wound covering materials that are popular at present include a polyurethane film, hydrocolloid dressing, polyurethane foam dressing, an alginate dressing, etc., and these products have an effect of retaining blood or exudate that bleeds out from a wound surface to thereby maintain the moist environment and promote epidermal cell division and migration.

### Citation List

### Non-Patent Literature

Non Patent Literature 1: Future Wound care, written by Makoto Natsui, Igaku-Shoin Ltd. (2003/08)

### Disclosure of Invention

### Problems to be Solved by the Invention

A wound covering material such as a polyurethane film, a hydrocolloid dressing, a polyurethane foam dressing, an alginate dressing or the like is effective for promoting epidermal cell division and migration by retaining exudate that bleeds out from a wound surface to thereby maintain the moist environment, and can draw to the utmost extent the radical natural healing ability that a patient has. However, for patients having a poor healing ability such as aged persons and others, in the case where wounds are bedsores, or where the volume and the area of the cut opening are large, or where a continuous force may be given to the wound surface, a relatively long period of time would be taken for wound closing, even though such a wound covering material is used in treatment. The increase in the healing time results in copious exudate bleeding or abnormal collagen production, therefore causing some risks of epidermal inflammation or scar formation.

Further, for diabetic patients, even though wound covering materials listed above are used, it may lead to delayed wound healing owing to immune function aberration, protein insufficiency, enzyme insufficiency or the like, and there may be a possibility or causing some serious complications by bacterial invasion from the wound surface.

Recently, the principle of not using a disinfectant for wound care has become changed. In detail, in the case where bacteria merely stay in a wound surface, or where growing bacteria do not have any influence on a host, disinfection is unnecessary. However, in the case where the number of bacteria has increased and wound infection would be developed, or where bacteria have invaded inside tissue and would do real damage to a host, use of a disinfectant is said to be necessary.

The present invention has been made in consideration of the above-mentioned situation, and its object is to provide a glass composition for wound care, which promotes a wound healing process by providing a moist environment and nutrients required for growth of epidermal cells and has bactericidal properties for preventing critical fixation of bacteria to a wound surface and infection with bacteria, and to provide a wound covering material that uses the glass composition.

### Means for Solving the Problems

A glass composition for wound care according to the present invention contains, by mass% in terms of oxides, 5 to 70% of SiO₂, 0 to 10% of Al₂O₃, 5 to 40.0% of B₂O₃, and 1 to 50% of CaO. Here, "for wound care" indicates use of the composition for wound healing in any and every form including healing by wound covering.

The composition for wound care according to the present invention having the above-mentioned constitution has biocompatibility. In addition, the composition can dissolve in the blood or exudate that bleeds out form a wound surface to thereby provide Ca (calcium) to be a nutrient for epidermal cells or B (boron) having a bactericidal effect against bacteria, to a wound surface.

In addition, preferably, the glass composition for wound care according to the present invention further contains, by mass% in terms of oxides, 0 to 20% of MgO, 0 to 20% of Na₂O, 0 to 40% of K₂O and 0 to 20% of P₂O₅.

In addition, in the glass composition for wound care according to the present invention, Na₂O + K₂O is preferably 5 to 40% by mass% in terms of oxides. Here, "Na₂O + K₂O" means a total content of Na₂O and K₂O.

In addition, also preferably, in the glass composition for wound care according the present invention, when a glass having a weight fraction of a density × 0.256, as classified to have a grain size of 300 to 500 µm, is subjected to an elution test of immersing the glass in 60 ml of a simulated body fluid at 37°C for 2 days with stirring once per day, a B concentration in the simulated body fluid is 0.1 mM or more and a Ca concentration therein is 3.8 mM or more.

Employing the above-mentioned constitution, the glass composition can sufficiently provide Ca and B necessary for wound healing to a wound surface.

Also preferably, in the glass composition for wound care according to the present invention, a liquidus viscosity is 10^{0.5} dPa·s or more. Here, "liquidus viscosity" is a value determined according to the following method. First, a bulky glass sample is ground to have a controlled grain size that falls within a range of 300 to 500 µm. Next, the ground glass sample is filled in a refractory container in a state having a suitable bulk density. Subsequently, the refractory container is put in an indirect-heating, temperature-gradient furnace and left therein statically, then kept heated in an air atmosphere for 16 hours, and the test specimen is taken out along with the refractory container from the temperature-gradient furnace, and cooled down to a room temperature. Subsequently, the glass sample is observed with an optical microscope and checked for the crystal deposition part therein, and from the temperature distribution information inside the temperature-gradient furnace, the crystal deposition temperature (liquidus temperature) is specified. Using the thus-determined crystal deposition temperature, the viscosity corresponding to the crystal deposition temperature is obtained from the viscosity curve that is prepared separately.

Employing the above-mentioned constitution, glass hardly devitrifies during molding and beads to mix in material can be reduced.

In addition, in the glass composition for wound care according to the present invention, a temperature corresponding to a viscosity of 10^{1.0} dPa·s is preferably 1500°C or lower. Here, "a temperature corresponding to a viscosity of 10^{1.0} dPa·s" is a value determined according to the following method. First, a bulky glass sample is ground to have a suitable size, and put into an alumina crucible with preventing gas bubbling therein as much as possible. Subsequently, the alumina crucible is heated to make the sample in a melt state, and the glass viscosities at different temperatures are measured according to a platinum ball pulling method. Subsequently, using the measured values of the glass viscosities, a constant of the Vogel-Fulcher equation is calculated to form a viscosity curve, and according to an interpolation method, a temperature to give a viscosity of 10^{1.0} dPa·s is calculated. The viscosity of 10^{1.0} dPa·s is a viscosity suitable for spinning according to a melt-blow method or the like, and in the present invention, the temperature corresponding to the viscosity is referred to as "a spinning temperature".

Employing the above-mentioned constitution, the lifetime of a noble metal-made spinning device for use in molding can be prolonged. In addition, the exchange frequency of the spinning device can be reduced, and therefore the production cost can be thereby reduced.

A wound covering material according to the present invention is a cottony body or a nonwoven fabric containing the above-mentioned glass composition. Here, "cottony body" indicates an amorphous fiber clump is three-dimensionally compressible owing to voids existing between the fibers. "Nonwoven fabric" indicates a cottony compressed body that has been molded in the form of a sheet or a fabric, in which a large number of fibers are irregularly entangled.

The wound covering material according to the present invention that has the above-mentioned constitution can retain the exudate that bleeds out from a wound surface while providing Ca to be a nutrient for epidermal cells or B having a bactericidal effect against bacteria, and therefore can provide a moist environment necessary for growth of epidermal cells.

In use for healing of a wound surface that bleeds much exudate, the wound covering material is preferably used in the form of a cottony body. When used in the form of a cottony body, the wound covering material can realize an increased water absorption and therefore can prevent skin maceration in an excessive moist environment. Further, a trouble of bleeding an exudate out of a wound hardly occurs. On the other hand, for use for healing of a wound surface that bleeds a little exudate, the wound covering material is preferably used in the form of a nonwoven fabric. When used in the form of a nonwoven fabric, the water absorption by the wound covering material reduces, therefore preventing a wound surface from drying.

In the wound covering material according to the present invention, an average fiber diameter of glass fibers that constitute the cottony body or the nonwoven fabric is preferably 100 nm to 10 µm. Here, "average fiber diameter of glass fibers" is determined according to a method where, using a scanning electron microscope (HITACHI s-3400N Type II), a secondary electron image or a reflected electron image of glass fibers is taken, and using the length measuring function of the scanning electron microscope, the diameter of 50 glass fibers is measured, and the measured values are averaged to give an average value to be the average fiber diameter.

Employing the above-mentioned constitution, the wound covering material can readily secure a sufficient dissolution amount in a body fluid while maintaining a sufficient form for retaining the blood or exudate.

In the wound covering material according to the present invention, preferably, glass beads are mixed in the cottony body or the nonwoven fabric, and the amount of glass beads mixed with is, by mass%, 50% or less of the entire cottony body or nonwoven fabric. In the present invention, "glass beads" indicate those of molten glass that have become nearly spherical forms owing to surface tension. Here, "amount of glass beads mixed with" can be determined by the method described as follows. The weight of the given cottony body or the nonwoven fabric is measured, the sample is put into a beaker, and then an alcohol is poured into the beaker and stirred for 3 minutes using, for example, a magnetic stirrer. After the stirring is stopped, waited for 20 seconds to precipitate glass beads, thereafter immediately the supernatant liquid except the precipitate is transferred into a different beaker. This operation is repeated, the collected precipitate is dried, then the weight of the precipitate is measured, and the weight ratio of the precipitate to the initial cottony body or the nonwoven fabric is calculated.

In producing a cottony body or a nonwoven fabric by fiberizing molten glass, a part of glass may form beads and may mix in the resultant cottony body or nonwoven fabric. As compared with fibers, beads may have a small specific surface area per unit mass, and therefore may tend to be difficult to dissolve in a body fluid or the like. Consequently, when the proportion of the amount of glass beads mixed with increases, a sufficient amount of Ca or B could hardly be eluted from the cottony body or the nonwoven fabric. However, in the case where the above-mentioned constitution is employed, the content of the glass fibers is large and therefore it is easy to fully secure the dissolution amount thereof in a body fluid. In addition, a rough feel of the cottony body or the nonwoven fabric can be relieved, therefore reducing risks of epidermal inflammation or scar formation. In addition, in placing into a wound surface, the pain of a patient can be relieved.

In the wound covering material according to the present invention, preferably, an average diameter of the glass beads is 500 µm or less. Here, "average diameter of glass beads" is determined according to a method where, using a length measuring function of an optical microscope, the diameter of 50 glass fibers is measured, and the measured values are averaged to give an average value to be the average diameter of glass beads.

Employing the above-mentioned constitution, the specific surface area of the glass beads can be large and a dissolution amount in a body fluid can be sufficiently secured. In addition, a rough feel of a cottony body or a nonwoven fabric can be relieved, therefore reducing risks of epidermal inflammation or scar formation. In addition, in placing into a wound surface, the pain of a patient can be relieved.

A method for producing a wound covering material according to the present invention includes melting a starting material batch that is prepared to give the above-mentioned glass composition in a glass melting furnace, and continuously ejecting the molten glass through a glass ejecting nozzle while applying an air jet to an area around the glass ejecting nozzle to thereby make the glass into a cottony form.

In the method of the present invention that has the above-mentioned constitution, a so-called melt blow process is employed where air is jetted around molten glass to blow and stretch the glass into a fibrous form, and therefore, a cotton-like, wound covering material with fibers entangled therein can be easy to produce.

In addition, according to the method of the present invention, glass having the above-mentioned characteristics is used, and therefore the wound healing effect of the resultant wound covering material is high.

In addition, the production method for the wound covering material according to the present invention includes forming a nonwoven fabric by compressing glass shaped in a cottony form.

### Embodiments for Carrying Out the Invention

Regarding a glass composition for wound care according to the present invention, the effects of the components constituting the glass and the reason why the content of each component is defined as above are described hereinunder. For the description of the content range of each component, the expression of % is by mass.

SiO₂ is a main component forming the network former of a glass. It is also a component of increasing the viscosity of glass. Further, it is a component of greatly lowering the melting speed of glass. The content of SiO₂ is 5 to 70%, preferably 5 to 50%, more preferably 8 to 45%, even more preferably 10 to 35%, still more preferably 10 to 30%, most preferably 10 to 25%. When the content of SiO₂ is too large, the dissolution speed of glass in blood or exudate may lower, and if so, therefore, the glass composition could hardly exhibit the effect for cell growth and the bactericidal effect necessary for a wound covering material. In addition, the spinning temperature (temperature corresponding to a viscosity of 10^{1.0} dPa·s) increases and the cost for production therefore increases. When the content of SiO₂ is too small, the viscosity of glass lowers and the liquidus viscosity extremely lowers, and therefore, if so, the amount of beads mixed with in forming into a cottony body or a nonwoven fabric increases. In addition, the mechanical strength of glass lowers and the cottony body is readily compressed and therefore could not secure necessary voids therein and, as a result, the retention amount of blood or exudate in the body lowers to hardly maintain a moist environment.

Al₂O₃ is a component that has an effect of preventing crystal precipitation or phase separation in molten glass. The content of Al₂O₃ is 0 to 10%, preferably 0 to 5%, more preferably 0 to 3%. When the content of Al₂O₃ is too large, the dissolution speed of glass in blood or exudate lowers. In addition, the spinning temperature increases and the cost for production therefore increases.

B₂O₃ is a component forming the network former of a glass, like SiO₂; however, this does not increase the glass melting temperature different from SiO₂, but rather acts to lower the melting temperature. In addition, as dissolving in blood or exudate, this component exhibits a bactericidal effect. Further, this is a component of remarkably increasing the glass dissolution speed. The content of B₂O₃ is 5 to 40.0%, preferably 10 to 39%, 15 to 35%, 19 to 30%, 19 to 27%, especially preferably 19 to 25%. When the content of B₂O₃ is too small, the glass composition could not exhibit a bactericidal effect of preventing critical fixation of bacteria to a wound surface and preventing bacterial infection. Further, the glass dissolution speed lowers and the glass composition could hardly realize an effect of cell growth necessary for a wound covering material and a bactericidal effect. When the content of B₂O₃ is too large, an excessive bactericidal effect would act on a wound surface to lower the wound healing speed. In addition, the glass viscosity may lower and the liquidus viscosity may lower, and therefore in the case where the wound covering material is produced according to a melt blow method or the like, the amount of beads mixed with may increase.

CaO is a component of lowering the viscosity of glass, and is a component that exhibits an effect of promoting cell growth when eluted in blood or exudate. The content of CaO is 1 to 50%, preferably 10 to 45%, more preferably 15 to 40%, even more preferably 18 8 to 38%, still more preferably 23 to 38%, and most preferably 28 to 38%. When the content of CaO is too small, the composition could hardly exhibit the effect of promoting cell growth. When the content of CaO is too large, the liquidus temperature increases and, when melted, the composition could hardly form homogeneous glass.

MgO is a component acting as a flux to facilitate melting of glass materials and is, at the same time, extremely effective for lowering the melting point of glass, and is therefore a component bettering foam breaking in melting glass to give homogeneous glass. The content of MgO is preferably 0 to 20%, more preferably 0 to 10%, even more preferably 0.5 to 8%. When the content of MgO is too large, the viscosity of glass lowers and the liquidus viscosity lowers, and therefore in the case where a wound coating material is produced according to a melt blow method or the like, the amount of beads mixed with increases. Further, the dissolution amount of Mg (magnesium) in blood or exudate increases, and relatively, therefore the Ca dissolution amount extremely lowers.

Na₂O and K₂O that are alkali metal oxides are components to lower the viscosity of glass and to enhance the meltability or formability thereof. The total content of Na₂O and K₂O (Na₂O + K₂O) is preferably 5 to 40%, more preferably 10 to 30%, even more preferably 15 to 25%. When (Na₂O + K₂O) is too small, the glass spinning temperature increases and the cost for production therefore increases. When (Na₂O + K₂O) is too large, the dissolution amount of Na (sodium) and K (potassium) in blood or exudate increases, and relatively, therefore, the dissolution amount of Ca greatly lowers. In addition, the glass viscosity lowers and the liquidus viscosity extremely lowers, and therefore in the case where the wound covering material is produced according to a melt blow method or the like, the amount of beads mixed with increases.

Na₂O is a component of enhancing the meltability and the formability of glass by lowering the viscosity of glass. The content of Na₂O is preferably 0 to 20%, more preferably 2 to 15%, even more preferably 4 to 10%. When the content of Na₂O is too large, the viscosity of glass lowers and the liquidus viscosity greatly lowers and therefore, in the case where the wound covering material is produced according to a melt blow method or the like, the amount of beads mixed with increases. Further, the dissolution amount ofNa in blood or exudate increases and relatively, therefore, the dissolution amount of Ca greatly lowers.

K₂O is a component of enhancing the meltability and the formability of glass by lowering the viscosity of glass. The content of K₂O is preferably 0 to 40%, more preferably 5 to 30%, even more preferably 7 to 20%, still more preferably 7 to 15%. When the content of K₂O is too large, the viscosity of glass lowers and the liquidus viscosity greatly lowers and therefore, in the case where the wound covering material is produced according to a melt blow method or the like, the amount of beads mixed with increases. Further, the dissolution amount of K in blood or exudate increases and relatively, therefore, the dissolution amount of Ca greatly lowers.

P₂O₅ is a component that vitrifies by itself to constitute the network structure of glass. The content of P₂O₅ is preferably 0 to 20%, more preferably 0 to 10%, even more preferably 0 to 5%. When the P₂O₅ content is too large, the dissolution speed of glass in blood or exudate lowers. Further, a reaction layer containing P (phosphorus) and Ca tends to form on the surface of molten glass to interfere with glass melting, and a part of Ca is consumed in forming the reaction layer to lower the dissolution amount of Ca.

In the glass composition for wound care of the present invention, the glass dissolution speed varies depending on the molar ratio of Si (silicon) and B (boron) (B/Si) in the glass composition. When the value of B/Si is larger, the proportion of B in the glass network increases more to lower chemical durability, and the glass dissolution speed thereby increases. Accordingly, for increasing the Ca dissolution amount to promote cell growth, it is advantageous that the value of B/Si is larger. However, for increasing the value of B/Si, the B₂O₃ content in glass must be increased, but if so, the dissolution amount of B increases and the composition may exhibit an excessive bactericidal effect.

The value of B/Si is preferably 0.1 to 20.0, more preferably 0.2 to 10.0, even more preferably 0.3 to 9.0, further more preferably 0.5 to 5.0, and most preferably 1.0 to 3.0. When the value of B/Si is too small, the composition could hardly exhibit the effect for cell growth and the bactericidal effect necessary for the wound covering material. On the other hand, when the value of B/Si is too large, the composition may exhibit an excessive bactericidal effect to interfere with cell growth.

The composition may contain any other component than the above-mentioned components (SiO₂, B₂O₃, CaO, MgO, Na₂O, K₂O, P₂O₅). However, desirably, the composition is so controlled that the total content of the above-mentioned component could be 98% or more, especially 99% or more. The reason is that, when the total content of these components is less than 98%, there may occur various disadvantages that the dissolution speed of Ca and B of glass in blood or exudate may lower owing to incorporation of unintended foreign components and the characteristics as the wound covering material may degrade, or the liquidus viscosity greatly lowers and the amount of beads mixes with increases and the biocompatibility worsens.

As the other components than the above-mentioned components, for example, the glass composition may contain minor components such as H₂, CO₂, CO, H₂O, He, Ne, Ar, N₂ and the like in an amount of up to 0.1% each. In glass, noble metal elements such as Pt, Rh, Au and the like may be contained in glass in an amount of up to 500 ppm.

Further, for enhancing the bactericidal effect, Cu, Ag, Zn, Sr, Ba, Fe, F, Mo, Au, Mn, Sn, Ce, Cl, La, W, Nb, Y and the like may be contained in a total amount of up to 2%.

Preferably, in the glass composition for wound care of the present invention, when the glass having a weight fraction of a density × 0.256, as classified to have a grain size of 300 to 500 µm, is subjected to an elution test of immersing it in 60 ml of a simulated body fluid at 37°C for 2 days with stirring once/day, the B concentration in the simulated body fluid is 0.1 mM or more and the Ca concentration therein is 3.8 mM or more. When the B concentration in the simulated body fluid in this elution test is less than 0.1 mM, or when the Ca concentration is less than 3.8 mM, the composition could hardly exhibit the effect of cell growth and the bactericidal effect necessary as a wound covering material.

Also preferably, in the glass composition for wound care of the present invention, the liquidus viscosity is 10^{0.5} dPa·s or more, 10^{0.8} dPa·s or more, 10^{1.0} dPa·s or more, 10^{1.2} dPa·s or more, especially 10^{1.4} dPa·s or more. When the liquidus viscosity is too low, the amount of glass beads to mix in during production of a cottony body or a nonwoven fabric by spinning of molten glass may increase.

In the glass composition for wound care of the present invention, the temperature (spinning temperature) corresponding to a viscosity of 10^{1.0} dPa·s is preferably 1500°C or lower, 1400°C or lower, 1300°C or lower, especially 1200°C or lower. When the spinning temperature is too high, high-temperature spinning is needed and, if so, the spinning device made of a noble metal may be seriously damaged and the device exchange frequency increases, that is, the production cost increases.

The wound covering material of the present invention is formed of the glass composition for wound care that has the above-mentioned composition and characteristics. More specifically, the wound covering material is an amorphous cottony body or nonwoven fabric where a large number of glass fibers having the above-mentioned composition and characteristics are irregularly entangled. The cottony body can be three-dimensionally compressed owing to the voids existing between fibers.

The glass fibers constituting the wound covering material may have an uneven fiber diameter, but the average fiber diameter thereof is preferably within a range of 100 nm to 10 µm, especially within a range of 500 to 5 µm. When the average fiber diameter is too small, the mechanical strength of the glass fibers lowers. Further, in the case where the composition is used in the form of a cottony body, the cottony body is readily compressed and therefore the blood or exudate retention amount therein may lower and the resultant body could hardly maintain a moist environment. When the average fiber diameter is too large, the specific surface area of the cottony body or the nonwoven fabric reduces, and therefore the glass dissolution speed lowers, that is, a sufficient amount of Ca and B could hardly be provided to blood or exudate, and the characteristics as a wound care material worsen.

Glass beads may be mixed in the cottony body or the nonwoven fabric to constitute the wound covering material. In this case, the proportion of the glass beads to the cottony body or the nonwoven fabric is, by mass%, preferably 50% or less, 40% or less, and especially 30% or less. When the proportion of the glass beads is too large, the specific surface area of the cottony body or the nonwoven fabric reduces and the glass dissolution speed lowers, that is, a sufficient amount of Ca and B could hardly be provided to blood or exudate, and the characteristics as a wound care material worsen. In addition, there is another risk that glass beads may stimulate skin to cause epidermal inflammation or scar formation. Further, in placing into a wound surface, the patient may suffer from pain.

Preferably, the average diameter of the glass beads is 500 µm or less, especially 100 µm or less. When the average diameter of the glass beads is too large, the specific surface area of the cottony body or the nonwoven fabric reduces and the glass dissolution rate lowers, that is, a sufficient amount of Ca and B could hardly be provided to blood or exudate, and the characteristics as a wound care material worsen. In addition, there is another risk that glass beads may stimulate skin to cause epidermal inflammation or scar formation. Further, in placing into a wound surface, the patient may suffer from pain.

The wound covering material of the present invention may contain any other various types of glass bodies such as powdery or flaky glass substances in addition to glass fibers and glass beads. Further, various chemicals may be added to or infiltrated in the cottony body or the nonwoven fabric.

Next, a method for producing the wound covering material of the present invention is described with reference to a melt blow method as an example thereof. The wound covering material of the present invention can be produced in any other method than a melt blow method. For example, a so-called electrospinning method where a high voltage is applied between a glass-ejecting nozzle and a target electrode arranged to face the nozzle member, and while the electrically-charged molten glass being ejected from the ejecting nozzle is attracted toward the electrode member side, the glass is formed into fibers, or a so-called rotational spinning process where molten glass is run down from a forehearth to be introduced into a spinner (rotor), and the spinner is rotated at a high speed to eject fibrous glass through the orifice arranged in the spinner side wall part is also employable.

First, glass materials are mixed to prepare glass having the above-mentioned composition. Glass cullet may be used as a part or all of the glass materials. The glass composition and the characteristics thereof are as described above, and description thereof is omitted here.

Next, the prepared glass material batch is put into a glass melting furnace, and vitrified, melted and homogenized. The melting temperature is preferably 1200 to 1600°C or so.

Subsequently, the molten glass is spun into glass fibers. In detail, the molten glass is introduced into a noble metal-made nozzle member equipped with ejecting nozzles for ejecting the molten glass. The molten glass thus introduced into the nozzle member runs down from one or more glass-ejecting nozzles arranged at the bottom of the member. High-speed air is jetted toward the thus-running down molten glass from one side, both sides or the whole circumference of the ejecting nozzles for stretching the glass melt to be formed into fibrous forms, thereby producing a wound covering material formed of cottony glass fibers. Further, the thus-produced cottony body is compressed to give a nonwoven fabric. For example, a method is employable where a cottony body is continuously deposited to have a uniform thickness on a conveyor having a metal net, and compressed with rollers. The inner diameter of the glass ejecting nozzle is preferably 2 mm or less, more preferably 1.5 mm or less, even more preferably 1.0 mm or less. The temperature of the high-speed air is preferably 500°C or more, more preferably 800°C or more, even more preferably 900°C or more. The characteristics of the wound covering material are as described above, and the description thereof is omitted here.

The glass composition for wound care of the present invention is not limited to use as a wound covering material described above. For example, the composition may be formed into powder, ribbons, flakes, hollow spheres or the like, and may be used in various applications. For example, the composition may be formed into a powder, mixed with an ointment such as vaseline, or with a cream or lotion to be applied to a wound surface.

### Examples

The present invention is described in detail with reference to Examples hereinunder.

Tables 1 to 5 show Examples of the present invention (Sample Nos. 1 to 17, 21 to 25) and Comparative Examples (Sample Nos. 18 to 20).

**[Table 1]**

| Sample No. | | 1 | 2 | 3 | 4 | 5 |
|---|---|---|---|---|---|---|
| Glass Composition (mass%) | SiO₂ | 15.3 | 32.30 | 20.58 | 44.25 | 32.92 |
| | Al₂O₃ | | | | | |
| | B₂O₃ | 24.6 | 27.28 | 37.94 | 7.35 | 17.94 |
| | MgO | 4.8 | 4.33 | 4.45 | 4.30 | 4.42 |
| | CaO | 36.9 | 17.25 | 17.70 | 24.88 | 25.52 |
| | Na₂O | 5.6 | 5.13 | 5.26 | 5.10 | 5.23 |
| | K₂O | 11.4 | 10.35 | 10.62 | 10.28 | 10.55 |
| | P₂O₅ | 1.4 | 3.36 | 3.45 | 3.34 | 3.43 |
| | Na₂O+K₂O | 17.0 | 15.48 | 15.88 | 15.37 | 15.78 |
| Molar Ratio of Boron to Silicon in Glass Composition (B/Si) | | 2.8 | 1.5 | 3.2 | 0.3 | 0.9 |
| B Concentration in Simulated body fluid (mM) | | 14.5 | 10.3 | 12.3 | 1.6 | 3.4 |
| Ca Concentration in Simulated body fluid (mM) | | 12.6 | 5.2 | 4.8 | 5.2 | 4.8 |
| Spinning Temperature (°C) | | 891 | 1084 | 991 | 1260 | 1067 |
| Liquidus viscosity (dPa·s) | | 10^{0.4} | 10^{1.0} | 10^{0.8} | 10^{1.7} | 10^{1.7} |
| Average Fiber Diameter (µm) | | 0.6 | Not measured | Not measured | 1.0 | Not measured |

**[Table 2]**

| Sample No. | | 6 | 7 | 8 | 9 | 10 |
|---|---|---|---|---|---|---|
| Glass Composition (mass%) | SiO₂ | 20.99 | 8.39 | 33.57 | 21.41 | 63.01 |
| | Al₂O₃ | | | | | |
| | B₂O₃ | 28.56 | 39.76 | 8.22 | 18.79 | 7.22 |
| | MgO | 4.54 | 4.66 | 4.50 | 4.63 | 3.95 |
| | CaO | 26.21 | 26.93 | 34.13 | 35.06 | 8.63 |
| | Na₂O | 5.37 | 5.52 | 5.33 | 5.48 | 4.68 |
| | K₂O | 10.83 | 11.13 | 10.75 | 11.05 | 9.44 |
| | P₂O₅ | 3.52 | 3.61 | 3.49 | 3.59 | 3.07 |
| | Na₂O+K₂O | 16.20 | 16.64 | 16.09 | 16.53 | 14.12 |
| Molar Ratio of Boron to Silicon in Glass Composition (B/Si) | | 2.4 | 8.2 | 0.4 | 1.5 | 0.2 |
| B Concentration in Simulated body fluid (mM) | | 9.3 | 21.5 | 0.8 | 3.2 | Not measured |
| Ca Concentration in Simulated body fluid (mM) | | 6.1 | 8.1 | 4.7 | 5.9 | Not measured |
| Spinning Temperature (°C) | | 955 | 879 | 1107 | 954 | Not measured |
| Liquidus viscosity (dPa·s) | | 10^{0.7} | 10^{0.9} | 10^{0.9} | 10^{0.8} | Not measured |
| Average Fiber Diameter (µm) | | Not measured | Not measured | Not measured | Not measured | Not measured |

**[Table 3]**

| Sample No. | | 11 | 12 | 13 | 14 | 15 |
|---|---|---|---|---|---|---|
| Glass Composition (mass%) | SiO₂ | 53.07 | 42.65 | 31.70 | 61.94 | 52.15 |
| | Al₂O₃ | | | | | |
| | B₂O₃ | 16.44 | 26.12 | 36.29 | 15.79 | 25.05 |
| | MgO | 4.05 | 4.15 | 4.25 | 3.89 | 3.98 |
| | CaO | 8.83 | 9.05 | 9.28 | 1.49 | 1.52 |
| | Na₂O | 4.79 | 4.91 | 5.03 | 4.60 | 4.71 |
| | K₂O | 9.67 | 9.91 | 10.15 | 9.28 | 9.50 |
| | P₂O₅ | 3.14 | 3.22 | 3.30 | 3.01 | 3.09 |
| | Na₂O+K₂O | 14.46 | 14.82 | 15.19 | 13.88 | 14.21 |
| Molar Ratio of Boron to Silicon in Glass Composition (B/Si) | | 0.5 | 1.1 | 2.0 | 0.4 | 0.8 |
| B Concentration in Simulated body fluid (mM) | | Not measured | Not measured | Not measured | Not measured | Not measured |
| Ca Concentration in Simulated body fluid (mM) | | Not measured | Not measured | Not measured | Not measured | Not measured |
| Spinning Temperature (°C) | | Not measured | Not measured | Not measured | Not measured | Not measured |
| Liquidus viscosity (dPa·s) | | Not measured | Not measured | Not measured | Not measured | Not measured |
| Average Fiber Diameter (µm) | | Not measured | Not measured | Not measured | Not measured | Not measured |

**[Table 4]**

| Sample No. | | 16 | 17 | 18 | 19 | 20 |
|---|---|---|---|---|---|---|
| Glass Composition (mass%) | SiO₂ | 41.89 | 31.45 | | 29.3 | 45.1 |
| | Al₂O₃ | | | | 13.8 | 13.8 |
| | B₂O₃ | 34.77 | 20.14 | 56.60 | 40.3 | 8.2 |
| | MgO | 4.07 | 5.07 | 4.60 | 2.1 | 2.1 |
| | CaO | 1.56 | 22.32 | 18.50 | 13.5 | 23.6 |
| | Na₂O | 4.82 | 5.55 | 5.50 | 0.6 | 6.8 |
| | K₂O | 9.73 | 12.27 | 11.10 | 0.2 | 0.2 |
| | P₂O₅ | 3.16 | 3.20 | 3.70 | 0.2 | 0.2 |
| | Na₂O+K₂O | 14.55 | 17.50 | 16.60 | 0.8 | 7.0 |
| Molar Ratio of Boron to Silicon in Glass Composition (B/Si) | | 1.4 | 1.1 | - | 2.4 | 0.3 |
| B Concentration in Simulated body fluid (mM) | | Not measured | 6.1 | 61.1 | 5.8 | 0.01 |
| Ca Concentration in Simulated body fluid (mM) | | Not measured | 5.1 | 10.4 | 3.6 | 2.6 |
| Spinning Temperature (°C) | | Not measured | 1106 | 965 | 1360 | 1463 |
| Liquidus viscosity (dPa·s) | | Not measured | 10^{1.4} | 10⁰⁴ | Not measured | Not measured |
| Average Fiber Diameter (µm) | | Not measured | 2.0 | 1.1 | 1.5 | 1.2 |

**[Table 5]**

| Sample No. | | 21 | 22 | 23 | 24 | 25 |
|---|---|---|---|---|---|---|
| Glass Composition (mass%) | SiO₂ | 14.77 | 21.20 | 14.92 | 15.08 | 14.96 |
| | Al₂O₃ | | | | | |
| | B₂O₃ | 34.08 | 23.72 | 29.24 | 24.29 | 24.11 |
| | MgO | 4.60 | 4.58 | 4.64 | 4.69 | 2.77 |
| | CaO | 26.56 | 30.59 | 31.01 | 35.55 | 37.92 |
| | Na₂O | 5.44 | 5.42 | 5.50 | 5.55 | 5.51 |
| | K₂O | 10.98 | 10.94 | 11.09 | 11.20 | 11.12 |
| | P₂O₅ | 3.56 | 3.55 | 3.60 | 3.64 | 3.61 |
| | Na₂O+K₂O | 16.42 | 16.36 | 16.58 | 16.75 | 16.63 |
| Molar Ratio of Boron to Silicon in Glass Composition (B/Si) | | 4.0 | 1.9 | 3.4 | 2.8 | 2.8 |
| B Concentration in Simulated body fluid (mM) | | 18.2 | 9.8 | 15.1 | 13.4 | 13.7 |
| Ca Concentration in Simulated body fluid (mM) | | 6.7 | 6.5 | 8.1 | 10.3 | 11.2 |
| Spinning Temperature (°C) | | 914 | 946 | 927 | 917 | 931 |
| Liquidus viscosity (dPa·s) | | 10^{0.6} | 10^{1.0} | 10^{0.5} | 10^{0.6} | 10^{0.4} |
| Average Fiber Diameter (µm) | | Not measured | Not measured | Not measured | 0.7 | Not measured |

The samples in Tables were prepared as follows.

First, various glass materials such as natural materials, chemical materials and others were weighed and mixed to have the glass composition shown in each Table, thereby preparing a glass batch. Next, the glass batch was put into a platinum-rhodium alloy-made crucible, and heated at 1200 to 1550°C for 4 hours in an indirect-heating electric furnace to give molten glass. For producing homogeneous molten metal, the molten glass was stirred plural times using a heat-resistant stirring rod at the time of heating. Subsequently, the resultant molten glass was cast into a heat-resistant mold, and left cooled in air to give a massive glass sample. Each resultant sample was tested in an elution test to measure the B concentration and the Ca concentration in a simulated body fluid. In addition, the spinning temperature and the liquidus temperature were measured.

Further, the massive glass sample was put into a noble metal-made pot equipped with a glass ejecting nozzle, and the glass sample was remelted by electrical heating. Subsequently, a high-speed air jet was applied to the glass flowing down from the ejecting nozzle so as to stretch and fiberize the molten glass thereby giving a cottony body. The average fiber diameter of the glass fibers constituting the thus-obtained cottony body was measured.

The elution test for measurement is as follows: First, the massive glass sample was ground, and the resultant grass fraction having a particle diameter of 300 to 500 µm was accurately weighed by a weight fraction of a density × 0.256, and subsequently, 60 ml of a simulated body fluid was put into a polypropylene container (PP container) having a volume of 100 ml, then the glass sample was dipped therein and subjected to an elution test under a condition of 37°C for 2 days. On this occasion, the sample was stirred once/day. For the stirring, the PP container was shaken a few times by hand. After the elution test, the test solution was filtered, and using ICP-OES, the B concentration and the Ca concentration in the eluate were quantified.

The simulated body fluid was prepared as follows: First, a beaker filled with 100 ml of distilled water was set on a stirrer. Next, reagents (7.995 g/L of NaCl, 0.353 g/L of NaHCO₃, 0.224 g/L of KCl, 0.174 g/L of K₂HPO₄, 0.305 g/L of MgCl₂·6H₂O, 0.368 g/L of CaCl₂·2H₂O, 0.071 g/L of Na₂SO₄) were weighed, and each reagent was sequentially added to and dissolved in the distilled water in such a manner that the next one is added after the first-added one has been completely dissolved, thereby preparing a solution. The reagent adhering to the powder paper was dissolved in the solution by spraying distilled water thereonto. Next, 90 ml of distilled water was added to 10 ml of 35% hydrochloric acid to prepare a diluted hydrochloric acid, and this was added to the solution little by little until the solution became noncloudy. Next, the solution was transferred to a 2-liter beaker, 825 ml of distilled water was added thereto, and stirred with a hot stirrer. Next, a pH meter was prepared, and diluted hydrochloric acid was gradually added through a dropper and dissolved to make the solution have a pH 2. Subsequently, 6.057 (g/L) of trishydroxymethylaminomethane (Tris-buffer) was dissolved in the solution to have a pH 8, and then, with heating with a hot stirrer, diluted hydrochloric acid was gradually added to finally give a solution having a pH or 7.25 at a liquid temperature 37°C. This solution was transferred to a plugged measuring cylinder, distilled water was added thereto to be 1 L and well shaken to mix the solution. The resultant solution was transferred to a plastic bottle, and then stored in a refrigerator for 1 day or more, thereby preparing the simulated body fluid for use in the experiment.

Regarding the theoretical value of the inorganic ion concentration of the simulated body fluid, Na⁺ is 142.0, K⁺ is 5.0, Mg²⁺ is 1.5, Ca²⁺ is 2.5, Cl⁻ is 148.8, and HPO⁴⁻ is1.0 (the unit is all mM).

The spinning temperature was measured as follows: First, the massive glass sample was ground into a suitable size, and put into an alumina-made crucible in such a manner that air-bubbling thereinto could be minimized. Subsequently, the alumina crucible was heated so that the sample therein could be in a molten state, and the glass viscosity at different temperatures was determined according to a platinum ball pulling method. Subsequently, a viscosity curve was drawn from the resultant plural measured values, and according to an interpolation method, a temperature to give 10^{1.0} dPa·s was calculated.

The liquidus temperature was measured as follows: First, the massive glass sample was ground to have a size falling within a range of 300 to 500 µm, and in that state, this was filled in a fireproof container to be in a state having a suitable bulk density. Subsequently, this fireproof container was put in an indirect-heating temperature-gradient furnace, statically left therein, and heated in air for 16 hours. Subsequently, the specimen was taken out along with the fireproof container from the temperature-gradient furnace, and cooled down to room temperature. Subsequently, the glass sample was observed with an optical microscope and checked for the crystal deposition part therein, and from the temperature distribution information inside the temperature-gradient furnace, the crystal deposition temperature (liquidus temperature) was specified. According to the method, the liquidus temperature was specified.

The average fiber diameter was measured as follows: First, using a scanning electron microscope (HITACHI s-3400N Type II), a secondary electron image or a reflected electron image of glass fibers was taken. Using the length measuring function of the scanning electron microscope, the diameter of 50 glass fibers was measured, and the measured values were averaged to give an average value to be the average fiber diameter.

## Claims

1. A glass composition for wound care comprising: by mass% in terms of oxides, 5 to 70% of SiO₂, 0 to 10% of Al₂O₃, 5 to 40.0% of B₂O₃, and 1 to 50% of CaO.

2. The glass composition for wound care according to claim 1, further comprising, by mass% in terms of oxides, 0 to 20% of MgO, 0 to 20% of Na₂O, 0 to 40% of K₂O and 0 to 20% of P₂O₅.

3. The glass composition for wound care according to claim 1 or 2, wherein Na₂O + K₂O is 5 to 40% by mass% in terms of oxides.

4. The glass composition for wound care according to any of clams 1 to 3, wherein, when a glass having a weight fraction of a density × 0.256, as classified to have a grain size of 300 to 500 µm, is subjected to an elution test of immersing the glass in 60 ml of a simulated body fluid at 37°C for 2 days with stirring once per day, a B concentration in the simulated body fluid is 0.1 mM or more and a Ca concentration in the simulated body fluid is 3.8 mM or more.

5. The glass composition for wound care according to any of clams 1 to 4, wherein a liquidus viscosity is 10^{0.5} dPa·s or more.

6. The glass composition for wound care according to any of clams 1 to 5, wherein a temperature corresponding to a viscosity of 10^{1.0} dPa·s is 1500°C or lower.

7. A wound covering material which is a cottony body or a nonwoven fabric comprising a glass composition of any one of claims 1 to 6.

8. The wound covering material according to claim 7, wherein an average fiber diameter of glass fibers constituting the cottony body or the nonwoven fabric is 100 nm to 10 µm.

9. The wound covering material according to claim 7 or 8, wherein glass beads are mixed in the cottony body or in the nonwoven fabric, and the amount of glass beads mixed with is, based on mass%, 50% or less of the entire cottony body.

10. The wound covering material according to claim 9, wherein an average diameter of the glass beads is 500 µm or less.

11. A method for producing a wound covering material, comprising: melting a starting material batch prepared to give a glass composition of any of claims 1 to 6 in a glass melting furnace; and continuously ejecting the molten glass through a glass ejecting nozzle while applying an air jet to an area around the glass ejecting nozzle to make the glass into a cottony form.

12. The method for producing a wound covering material according to claim 11, further comprising forming a nonwoven fabric by compressing the glass shaped in a cottony form.
